# FASCICULE DE BREVET EUROPEEN

(11) **EP 3 454 875 B1**
(45) Date de publication et mention de la délivrance du brevet: **23.09.2020**
(21) Numéro de dépôt: 17722071.2
(22) Date de dépôt: 12.05.2017
(51) Int. Cl.: A61K 36/37, C12N 5/04, A61P 17/06

(54) **PROCEDE DE PRODUCTION DU CELASTROL ET DE DERIVES TRITERPENIQUES PENTACYCLIQUES**
VERFAHREN ZUR HERSTELLUNG VON CELASTROL UND PENTACYCLISCHEN TRITERPENDERIVATEN
METHOD FOR PRODUCING CELASTROL AND PENTACYCLIC TRITERPENE DERIVATIVES

(30) Priorité: 12.05.2016 FR 1654240
(43) Date de publication de la demande: 20.03.2019
(73) Titulaire: Pierre Fabre Dermo-Cosmétique, 92100 Boulogne-Billancourt (FR)
(72) Inventeur: NGUYEN, Thien, 31180 Rouffiac-Tolosan (FR); COUSY, Adrien, 31870 Beaumont Sur Leze (FR); STEWARD, Nicolas, 31860 Pins-Justaret (FR)
(74) Mandataire: Regimbeau
(86) Numéro de dépôt international: PCT/EP2017/061504
(87) Numéro de publication internationale: WO 2017/194757

(56) Documents cités:
- FR-A1- 2 952 072
- DATABASE MEDLINE [Online] US NATIONAL LIBRARY OF MEDICINE (NLM), BETHESDA, MD, US; mai 2015 (2015-05), LI YAN ET AL: "[Effects of elicitors on growth of adventitious roots and contents of secondary metabolites in Tripterygium wilfordii Hook. f].", XP002765741, Database accession no. NLM26571694 & SHENG WU GONG CHENG XUE BAO = CHINESE JOURNAL OF BIOTECHNOLOGY MAY 2015, vol. 31, no. 5, mai 2015 (2015-05), pages 734-743, ISSN: 1000-3061
- RADHAMANI KANNAIYAN ET AL: "Molecular targets of celastrol derived from Thunder of God Vine: Potential role in the treatment of inflammatory disorders and cancer", CANCER LETTERS, NEW YORK, NY, US, vol. 303, no. 1, 28 octobre 2010 (2010-10-28), pages 9-20, XP028152697, ISSN: 0304-3835, DOI: 10.1016/J.CANLET.2010.10.025 [extrait le 2010-11-01]
- BRIAN ASTRY ET AL: "Celastrol, a Chinese herbal compound, controls autoimmune inflammation by altering the balance of pathogenic and regulatory T cells in the target organ", CLINICAL IMMUNOLOGY, vol. 157, no. 2, 1 avril 2015 (2015-04-01) , pages 228-238, XP055332621, US ISSN: 1521-6616, DOI: 10.1016/j.clim.2015.01.011
- YU-JIA LIU ET AL: "The MVA pathway genes expressions and accumulation of celastrol in Tripterygium wilfordii suspension cells in response to methyl jasmonate treatment", JOURNAL OF ASIAN NATURAL PRODUCTS RESEARCH, vol. 18, no. 7, 19 janvier 2016 (2016-01-19), pages 619-628, XP055333136, CH ISSN: 1028-6020, DOI: 10.1080/10286020.2015.1134504
- Guizhi Fan ET AL: "Chitosan activates defense responses and triterpenoid production in cell suspension cultures of Betula platyphylla Suk", African Journal of Biotechnology, 10 mai 2010 (2010-05-10), pages 2816-2820, XP055333769, DOI: 10.5897/AJB09.1975 Extrait de l'Internet: URL:http://www.academicjournals.org/journa l/AJB/article-full-text-pdf/FE4317F20888 [extrait le 2017-01-10]
- KUTNEY J P ET AL: "ANTI-INFLAMMATORY OLEANANE TRITERPENES FROM TRIPTERYGIUM WILFORDII CELL SUSPENSION CULTURES BY FUNGAL ELICITATION", PLANT CELL REPORTS, SPRINGER INTERNATIONAL, DE, vol. 12, 1 janvier 1993 (1993-01-01), pages 356-359, XP001026846, ISSN: 0721-7714, DOI: 10.1007/BF00237435
- KARLA RAMIREZ-ESTRADA ET AL: "Elicitation, an Effective Strategy for the Biotechnological Production of Bioactive High-Added Value Compounds in Plant Cell Factories", MOLECULES, vol. 21, no. 2, 3 février 2016 (2016-02-03), page 182, XP055333154, DOI: 10.3390/molecules21020182

## Description

La présente invention concerne un procédé de production d'un extrait brut enrichi en triterpènes pentacycliques, notamment en Célastrol, à partir d'une culture de cellules végétales (CCV) en suspension d'une plante de la famille des Celastraceae.

*Tripterygium wilfordii* est une plante médicinale, appartenant à la famille des Celastraceae, utilisée depuis des siècles en Asie du Sud-Est (dont la Chine) pour soigner les troubles inflammatoires, les maladies auto-immunes, et plus récemment le cancer. Les terpènes sont parmi les composants les plus actifs de la plante et sont localisés majoritairement dans les racines de la plante. Parmi eux, un triterpène pentacyclique de type norfriedelane, le Célastrol, est évalué pour son efficacité dans le traitement de l'obésité, de l'arthrite rhumatoïde, de la maladie de Crohn et du cancer de la prostate. Les besoins en approvisionnement en Célastrol sont donc en constante augmentation.

La dermatose inflammatoire, notamment le psoriasis ou la dermatite atopique, a des origines ou des causes multifactorielles. Les raisons de ces maladies dites auto-immunes ne sont pas encore complètement élucidées mais seraient liées à une dérégulation du système immunitaire notamment au niveau des réponses cellulaires. On peut classer les réponses cellulaires en 3 catégories : Th1, Th2 et récemment Th17. Bien que le psoriasis et la dermatite atopique présentent des différences au niveau des signes cliniques, elles présenteraient des ressemblances notamment au niveau de l'expression d'IL17 et IL22 caractéristiques de la réponse Th17 (Miyagaki *et al.* 2015). IL17 et IL22 seraient donc des cibles intéressantes pour traiter ces maladies. Ainsi des anticorps monoclonaux dirigés contre IL17, IL17R, IL22, IL23, TNF alpha ont été développés pour bloquer la voie Th17 (Lowes *et al.* 2014). Ces molécules sont efficaces pour le traitement du psoriasis, mais présentent de nombreux effets secondaires et induisent un coût de traitement exorbitant.

Par ailleurs, il est également connu que la voie Th17 est fortement activée dans les lésions de patients présentant de l'acné (Kelhala *et al.* 2014).

Le Célastrol est couramment obtenu par des procédés d'extraction végétale à partir des plantes de la famille de Celastraceae impliquant l'utilisation de solvants. Ces modes de biosynthèse impliquent des cycles de huit à dix ans pour qu'une jeune pousse de plante atteigne la maturité nécessaire pour être sacrifiée. En outre, l'utilisation de produits phytosanitaires rend le processus coûteux : des contaminants externes (métaux lourds) peuvent s'accumuler et l'extrait brut généré contient de multiples métabolites nécessitant plusieurs étapes de purification de cet extrait végétal afin d'isoler la fraction triterpénique. Par ailleurs, ces procédés ont des rendements faibles en Célastrol.

La synthèse chimique totale du Célastrol est possible (Camelio *et al.* 2015) mais nécessite une vingtaine d'étapes, ce qui augmente considérablement le coût final d'obtention du produit.

Une solution alternative a été d'obtenir du Célastrol à partir de cultures cellulaires en suspension de cellules souches générées à partir des racines ou des feuilles de la plante. Récemment, l'équipe de Coppede (Coppede *et al.* 2014) a pu obtenir à partir d'une culture de cellules de *M. ilicifolia* une quantité de Célastrol supérieure à celle obtenue par extraction classique à partir de la plante (0,304 mg de Célastrol par g de matières sèches, concentration maximale obtenue au bout de 8 jours de culture, soit 8,85 fois meilleure que par la méthode d'extraction classique). L'équipe de Liu *et al.* (Liu *et al.* 2016) a par ailleurs montré qu'en ajoutant du MeJa (jasmonate de méthyle) (de l'ordre de 50 µM) dans une culture de cellules végétales de *T. wilfordii,* la quantité de Célastrol se trouve augmentée de 4,82 fois par rapport à celle d'une culture sans MeJa : la concentration en mg de Célastrol par g de matière sèche est de 0,752 mg/g (culture avec MeJa) versus 0,154 mg/g (culture sans MeJa).

Ces procédés permettent d'obtenir des rendements en Célastrol améliorés. Cependant il existe toujours un véritable besoin de procédés viables économiquement et permettant d'obtenir rapidement du Célastrol en grande quantité.

La présente invention fournit un procédé de production d'un extrait brut enrichi en Célastrol à partir de cellules végétales d'une plante de la famille des Celastraceae, Tripterygium wilfordii.

En effet, les inventeurs ont mis au point un procédé permettant d'augmenter de manière surprenante le rendement en Célastrol dans la culture de cellules végétales d'une lignée de *T. wilfordii* en utilisant et en comparant diverses combinaisons d'éliciteurs. Les inventeurs ont en effet notamment observé que la division cellulaire et la production de Célastrol ne sont pas concomitantes. De manière surprenante, elles sont même incompatibles. Pour résoudre ce problème, les inventeurs ont mis au point un procédé comprenant une étape de prolifération cellulaire et une étape d'élicitation avec un cocktail d'agents éliciteurs qui stoppe la division cellulaire. Les inventeurs ont en outre mis en évidence que l'utilisation de couples d'éliciteurs, notamment le jasmonate de méthyle (MeJa) et la chitine, permet d'obtenir une concentration de **16,6 mg/g** de Célastrol par poids sec en fin de culture. Le rendement en Célastrol de cette culture élicitée est alors **22 fois** plus élevé par rapport à celui obtenu par Liu *et al.* (op. cit.). Le rendement par le procédé selon l'invention est par ailleurs **57 fois** plus élevé que celui obtenu par Coppede *et al.* (op. cit.) sans élicitation. Le procédé selon l'invention permet en outre d'enrichir en dérivés du Célastrol, notamment de type tripterpène pentacyclique, tels que la Tingénine A (encore appelée Tingénone ou Mayténine), la Tingénine B (encore appelée 22béta-Hydroxy-tingénone), la Pristimérine et la Tripterygone.

Par ailleurs, les inventeurs ont montré de façon surprenante qu'un extrait brut enrichi en triterpènes pentacycliques obtenu par ce procédé inhibe avec un effet dose les interleukines spécifiques du Th17 induites dans des cellules TCD4+ humaines.

La présente invention concerne donc un procédé de production d'un extrait brut enrichi en triterpènes pentacycliques comprenant les étapes suivantes :
(i) une phase de prolifération de cellules d'une plante de la famille des Celastraceae qui est Tripterygium wilfordii dans un milieu de prolifération,
(ii) une phase d'élicitation par ajout d'un cocktail d'élicitation à la culture cellulaire obtenue à l'étape (i), ledit cocktail d'élicitation comprenant au moins un éliciteur de type composé monocarboxylique qui est le jasmonate de méthyle et au moins un éliciteur biotique, qui est la chitine et
(iii) la préparation d'un extrait brut enrichi en tripterpènes pentacycliques à partir de la culture cellulaire obtenue à l'étape (ii).

Par ailleurs, la présente invention concerne un extrait susceptible d'être obtenu par le procédé selon l'invention, ainsi que ses utilisations.

Par « triterpènes pentacycliques » selon l'invention, on entend les triterpènes pentacycliques naturellement produits par les cellules d'une plante de la famille des Celastraceae et notamment le Célastrol (de formule I), la Tingénine A (encore appelée Tingénone ou Mayténine) (de formule II), la Tingénine B (encore appelée 22béta-Hydroxy-tingénone) (de formule III), la Pristimérine (de formule IV) et la Tripterygone (de formule V), notamment le Célastrol, la Tingénine A et la Tingénine B, notamment le Célastrol.

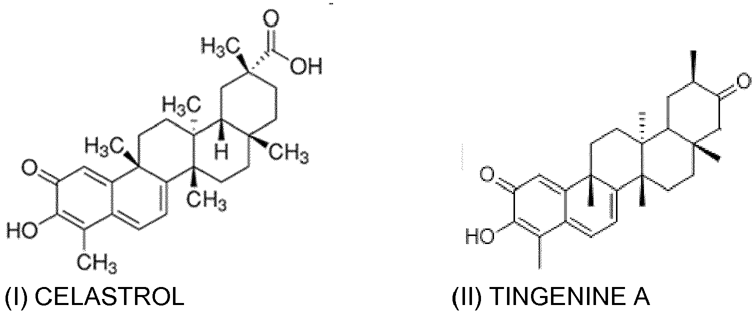

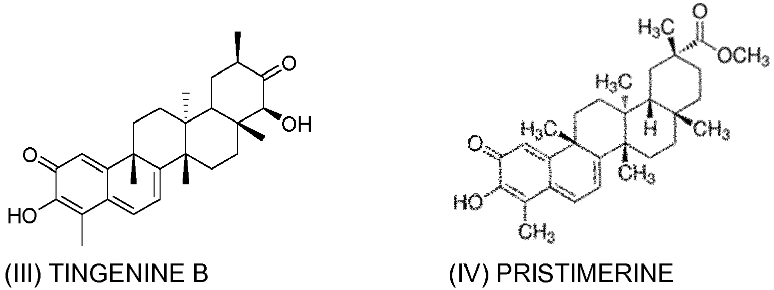

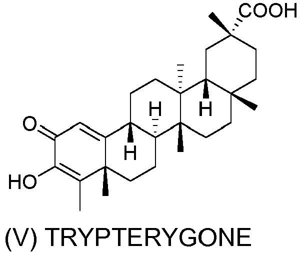

Par « extrait brut enrichi en triterpènes pentacycliques » selon l'invention, on entend un extrait brut enrichi comprenant une quantité de triterpènes pentacycliques au moins 2 fois, notamment au moins 5 fois, notamment au moins 10 fois, notamment entre 10 et 100 fois supérieure à celle obtenue par un procédé en l'absence d'une phase d'élicitation selon l'invention.

Par « extrait brut enrichi en Célastrol » selon l'invention, on entend un extrait brut enrichi comprenant une quantité de Célastrol au moins 2 fois, notamment au moins 5 fois, notamment au moins 10 fois, notamment entre 10 et 60 fois, notamment entre 10 et 20 fois supérieure à celle obtenue par un procédé en l'absence d'une phase d'élicitation selon l'invention.

Par « plante de la famille des Celastraceae » on entend l'ensemble des plantes appartenant à cette famille, et notamment les plantes du genre Tripterygium, Bhesa, Kokkona, Catha, Euonymus, Orthosphenia, Dicarpellum, Celastrus, Maytenus, Peritassa et Rzedowskia, notamment Tripterygium et Celastrus. Parmi le genre Tripterygium, on pourra notamment citer les espèces *Tripterygium wilfordii, Tripterygium regeli, Tripterygium hypoglaucum* ou *Tripterygium articulata*, notamment *Tripterygium wilfordii.*

Par « cellules d'une plante de la famille des Celastraceae » selon l'invention, on entend les cellules de n'importe quelle partie de la plante : graines, racines, parties aériennes, et notamment des parties aériennes.

Par « parties aériennes » selon l'invention, on entend les parties de la plante situées au-dessus du sol, par exemple, les feuilles, les tiges, les pétioles et/ou les inflorescences, notamment les feuilles.

Par « phase de prolifération de cellules d'une plante de la famille des Celastraceae », on entend selon l'invention une phase dans laquelle les cellules d'une plante de la famille des Celastraceae sont en suspension dans un milieu de prolifération et dans des conditions adaptées à leur prolifération. Ces cellules pourront notamment être obtenues à partir de cals avant leur mise en suspension. Si nécessaire, les suspensions cellulaires pourront être régulièrement réensemencées afin de les maintenir dans des conditions de prolifération.

Par « cal » selon l'invention, on entend un amas de cellules dédifférenciées, également appelées cellules souches ou cellules méristématiques.

L'induction de cals pourra être obtenue par toute méthode connue de l'homme du métier. Les cals selon l'invention pourront notamment être obtenues de la manière décrite ci-après.

L'induction de cals à partir d'un explant de tissu de partie de plante, notamment une partie aérienne, de la famille des Celastraceae, qui est *Tripterygium wilfordii,* est bien connue de l'homme du métier.

L'induction de cals pourra notamment être réalisée par :
- obtention d'un explant de tissu de la plante, par exemple un morceau de feuille de taille de 1 cm² environ,
- mise en culture de l'explant sur un milieu de prolifération selon l'invention gélosé (par exemple par ajout de 4 à 12 g/L d'agar, par exemple environ 8 g/L d'agar, au milieu de prolifération selon l'invention),
- incubation, notamment à l'obscurité, à une température d'environ 25-30°C, par exemple à environ 27°C à 28°C.

### Etape (i) : phase de prolifération de cellules

L'homme du métier connaissant les cultures de cellules d'une plante de la famille des Celatraceae pourra facilement déterminer la composition du milieu de prolifération nécessaire à leur prolifération. Préférentiellement, ce milieu de prolifération permettra la prolifération des cellules sous formes dédifférenciées, c'est-à-dire sous formes totipotentes. Le maintien sous forme dédifférenciée pourra notamment être obtenu par l'utilisation de ratios particuliers cytokinines/auxines dans le milieu de prolifération.

Le « milieu de prolifération » selon l'invention pourra notamment comprendre :
- au moins un macroélément, notamment choisi parmi NH₄NO₃, KNO₃, CaCl₂.2H₂O, MgSO₄.7H₂O, KH₂PO₄ et un mélange de ceux-ci, par exemple à une concentration en macroéléments totale comprise entre 1000 et 9000 mg/L, par exemple entre 3000 et 8000 mg/L de milieu de prolifération : lors de la phase de prolifération, la concentration en macroéléments totale du milieu de prolifération sera notamment comprise entre 3000 et 5500 mg/L de milieu prolifération ;
- au moins un microélément, notamment choisi parmi KI, H₃BO₃, MnSO4.4H₂O, ZnSO4.H₂O, Na₂MoO₄.2H₂O, CuSO₄.5H₂O, CoCl₂.6H₂O, FeSO₄.7H₂O, Na₂EDTA.2H₂O et un mélange de ceux-ci, par exemple à une concentration en microéléments totale comprise entre 10 et 200 mg/L, notamment entre 50 et 150 mg/L de milieu prolifération ;
- au moins une vitamine, notamment choisie parmi le myo-inositol, l'acide nicotinique, la pyridoxine-HCl, la thiamine-HCI et un mélange de ceux-ci, par exemple à une concentration en vitamines totale pouvant aller de 0,01 à 3 g/L, notamment de 0,05 à 1 g/L de milieu de prolifération ;
- au moins un acide aminé, notamment la glycine, par exemple à une concentration en acide aminés totale pouvant aller de 0,15 à 5 mg/L, notamment entre 1 et 4 mg/L de milieu de prolifération : lors de la phase de prolifération, la concentration en acide aminés du milieu de prolifération sera notamment comprise entre 1 et 2,5 mg/L de milieu prolifération ;
- au moins une source de carbone, notamment du saccharose, par exemple à une concentration totale en source de carbone de 10 à 70 g/L de milieu de prolifération, par exemple à environ 30 g/L ;
- au moins une hormone végétale (encore appelée hormone de croissance végétale ou facteur de croissance végétale ou régulateur de croissance végétale) notamment choisie parmi une ou plusieurs cytokinine, notamment la kinétine et/ou la 6-furfurylaminopurine, une ou plusieurs auxines, notamment l'acide 2,4-dichlorophénoxyacétique (2,4D) et/ou l'acide naphthalène acétique (NAA), et un mélange de celles-ci. Lors de la phase de prolfération le milieu de prolifération comprendra notamment au moins une cytokinine et au moins une auxine.

Les hormones de croissance végétale seront notamment ajoutées au milieu de prolifération à une concentration et à un ratio permettant la prolifération des cellules sous formes dédifférenciées. Elles seront notamment choisies parmi la kinétine, la 6-furfurylaminopurine, l'acide 2,4-dichlorophénoxyacétique (2,4D), l'acide naphthalène acétique (NAA) et un mélange de ceux-ci ; notamment choisies parmi la kinétine, l'acide 2,4-dichlorophénoxyacétique (2,4D), l'acide naphthalène acétique (NAA) et un mélange de ceux-ci. Il pourra s'agir en particulier d'un mélange de kinétine, acide 2,4-dichlorophénoxyacétique (2,4D) et acide naphthalène acétique.

La concentration en auxines selon l'invention sera notamment comprise entre 0,001 et 10 mg/L de milieu de prolifération, par exemple entre 0,1 et 3 mg/L de milieu de prolifération.

La concentration en cytokinines selon l'invention sera notamment comprise entre 0,01 et 0,5 mg/L de milieu de prolifération, notamment entre 0,05 et 0,15 mg/L.

Dans un mode de réalisation, le ratio hormonal auxines / cytokinines sera compris entre 0,2 à 2,5/0,01 à 0,5, notamment entre 1 à 2/ 0,05 à 0,2 notamment sera d'environ 1,5/0,1.

Notamment le milieu de de prolifération selon l'invention pourra comprendre 1,5 mg/L d'auxine, notamment d'acide 2,4-dichlorophénoxyacétique (2,4D) et d'acide naphthalène acétique (NAA), et 0,1 mg/L de cytokinine, notamment de kinétine.

Le milieu de prolifération sera stérile et préférentiellement à un pH proche de la neutralité.

Un exemple de milieu de prolifération adapté à la prolifération des cellules d'une plante de la famille des Celastraceae selon l'invention est notamment décrit par Murashige & Skoog (1962) ou dans les exemples de la présente demande.

Ce milieu de prolifération peut par exemple avoir la composition suivante (les concentrations sont exprimées par rapport au volume de milieu de prolifération sans cellule): Macroéléments: NH₄NO₃ à 1650 mg/L, KNO₃ à 1900 mg/L, CaCl₂.2H₂O à 440 mg/L, MgSO₄.7H₂O à 370 mg/L, KH₂PO₄ à 170 mg/L ; Microéléments : KI à 0,83 mg/L, H₃BO₃ à 6,2 mg/L, MnSO₄.4H₂O à 22,3 mg/L, ZnSO₄.H₂O à 6,6 mg/L, Na₂MoO₄.2H₂O à 0,25 mg/L, CuSO₄.5H₂O à 0,025 mg/L, CoCl₂.6H₂O à 0,025 mg/L, FeSO₄.7H₂O à 27,8 mg/L, Na₂EDTA.2H₂O à 37,3 mg/L ; Vitamines: myo-inositol à 100 mg/L, acide nicotinique à 0,5 mg/L, pyridoxine-HCl à 0,5 mg/L, thiamine-HCl à 0,5 mg/L ; Acides aminés : glycine à 2 mg/L ; Source de carbone : saccharose à 30 g/L ; et Hormones végétales : acide naphtalène acétique à 1 mg/L, acide 2,4-dichlorophénoxyacétique à 0,5 mg/L, kinétine à 0,1 mg/L, le tout ajusté à pH 6 avant stérilisation (par exemple par autoclavage de 20 min à 121°C ou par filtration sur filtre de 0,2 µm).

Alternativement, le milieu de prolifération peut avoir la composition suivante : (les concentrations sont exprimées par rapport au volume de milieu de prolifération sans cellule): Macroéléments: NH₄NO₃ à 1650 mg/L, KNO₃ à 2500 mg/L, CaCl₂.2H₂O à 440 mg/L, MgSO₄.7H₂O à 370 mg/L, KH₂PO₄ à 130 mg/L ; Microéléments : KI à 0,41 mg/L, H₃BO₃ à 6,2 mg/L, MnSO₄.4H₂O à 22,3 mg/L, ZnSO₄.H₂O à 7,5 mg/L, Na₂MoO₄.2H₂O à 0,25 mg/L, CuSO₄.5H₂O à 0,025 mg/L, CoCl₂.6H₂O à 0,025 mg/L, FeSO₄.7H₂O à 19,85 mg/L, Na₂EDTA.2H₂O à 26,64 mg/L ; Vitamines : myo-inositol à 50 mg/L, acide nicotinique à 0,25 mg/L, pyridoxine-HCI à 0,25 mg/L, thiamine-HCl à 0,25 mg/L ; Source de carbone : saccharose à 30 g/L ; et Hormones végétales : kinétine à 0,083 mg/L, acide 2,4-dichlorophénoxyacétique (2,4D) à 0,575 mg/L, acide naphthalène acétique (NAA) à 0,350 mg/L.

Alternativement, le milieu de prolifération selon l'invention peut avoir la composition suivante (les concentrations sont exprimées par rapport au volume de milieu de prolifération sans cellule): NH₄NO₃ à 20 mM, KNO₃ à 19 mM, CaCl₂.2H₂O à 3 mM, MgSO₄.7H₂O à 1,50 mM, KH₂PO₄ à 1,2 mM, KI à 0,005 mM, H₃BO₃ à 0,1 mM, MnSO₄.4H₂O à 0,1 mM, ZnSO₄.H₂O à 0,04 mM, Na₂MoO₄.2H₂O à 0,001 mM CuSO₄.5H₂O à 0,0001 mM, CoCl₂.6H₂O à 0,0001 mM, FeSO₄.7H₂O à 0,1 mM, Na₂EDTA.2H₂O à 0,1 mM, myo-inositol 0,5 mM, acide nicotinique à 0,004 mM, pyridoxine-HCI à 0,002 mM, thiamine-HCl à 0,0015 mM, glycine à 0,03 mM, saccharose à 87,6 mM, acide naphtalène acétique à 0,005 mM, acide 2,4-dichlorophénoxyacétique à 0,002 mM, et kinétine à 0,0005 mM.

L'ensemencement du milieu de prolifération pourra être réalisé à partir de la mise en suspension de cellules de cals à une concentration comprise entre 20 et 300 g dans 1 L de milieu de prolifération et préférentiellement entre 100 et 200 g dans 1L de milieu de prolifération, par exemple environ 150 g dans 1 L de milieu de prolifération.

La phase de prolifération aura lieu dans des conditions de multiplication de la biomasse. Par « conditions de multiplication de la biomasse » selon l'invention, on entend notamment les conditions de température, de durée, d'agitation, et de luminosité nécessaires à la prolifération des cellules en suspension. L'homme du métier connaissant les cultures de cellules d'une plante de la famille de Celatraceae pourra facilement déterminer les conditions de multiplication de la biomasse. Dans un mode de réalisation selon l'invention, l'étape de prolifération de la biomasse se fera à l'obscurité, à une température comprise entre 20 et 35°C, notamment entre 27 et 28°C, notamment à environ 27 ou 28°C, en particulier sous une agitation comprise entre 100 et 200 rpm, notamment à environ 125 rpm (orbitale de 22,5 mm) et pendant une durée comprise entre 10 et 30 jours, notamment 15 jours de culture.

Au cours de cette étape, les cellules peuvent être « repiquées » ou propagées, par exemple tous les 7 à 15 jours. Le repiquage des cellules est bien connu par l'homme du métier, il pourra notamment consister à diluer une partie de la culture cellulaire dans du milieu neuf concentré. Par exemple, 1/5^{ème} de la culture est remise en suspension dans un volume de milieu neuf correspondant au volume de la culture initiale. Il permet l'entretien de la lignée cellulaire en milieu liquide dans un état de prolifération.

### Etape (ii) - phase d'élicitation

Après la phase de prolifération, les cellules obtenues dans la phase (i) sont élicitées par ajout d'un cocktail d'élicitation. Le milieu de prolifération auquel a été ajouté le cocktail d'élicitation sera nommé « milieu d'élicitation » selon la présente invention.

La phase d'élicitation est la phase dans laquelle les cellules sont maintenues dans un état physiologique favorisant la biosynthèse de métabolites secondaires tels que les triterpènes pentacycliques.

La production de triterpènes pentacycliques, notamment de Célastrol, a lieu, au cours de la phase d'élicitation, dans le cytosol de la cellule et peut diffuser en partie dans le milieu d'élicitation. La phase d'élicitation au sens de la présente invention correspond donc à la phase de production (biosynthèse) du Célastrol et de ses dérivés (triterpènes pentacycliques).

L'élicitation se fera préférentiellement après une phase de prolifération de 7 à 21 jours, notamment 12 à 20 jours, notamment 15, 16 ou 17 jours (notamment sans repiquage). Alternativement l'ajout du cocktail d'élicitation pourra se faire lorsque la concentration en cellules obtenue lors de la phase de prolifération est double, notamment supérieure au double, par rapport à la concentration initiale en cellules dans le milieu de prolifération. L'ajout du cocktail d'élicitation pourra notamment se faire lorsque la concentration en cellule est supérieure à 200 g/L, par exemple entre 200 et 400 g/L, notamment environ 300 g/L (en quantité de cellules par litre de milieu de prolifération). Dans un mode de réalisation, l'ajout du cocktail d'élicitation se fera dans une culture dont la concentration en cellules est passée de 150 à 200 g/L lors du début de la phase de prolifération à une concentration comprise entre 300 et 400 g/L lors de la fin de la phase de prolifération.

A la suite de la phase de prolifération, les cellules végétales ont consommé la plupart des, voire tous les éléments contenus dans le milieu de prolifération, et en particulier les sources de carbone telles que le saccharose. Il peut donc être nécessaire de rétablir la composition du milieu avant ou en même temps que l'ajout du cocktail d'élicitation.

Pour rétablir la composition du milieu, on peut notamment concentrer la culture cellulaire obtenue après l'étape de prolifération, par exemple par décantation ou filtration puis ajouter du milieu de prolifération neuf aux cellules ainsi obtenues. Dans ce cas, les cellules seront re-suspendues dans le milieu de prolifération neuf de manière à obtenir une concentration comprise entre 200 g/L et 400 g/L, par exemple entre 250 et 350 g/L, notamment d'environ 300 g/L (en quantité de cellules par litre de milieu de prolifération).

Alternativement, on peut ajouter dans la culture cellulaire un mélange concentré permettant de rétablir les concentrations des éléments du milieu de prolifération. Le mélange concentré sera notamment ajouté juste avant, juste après ou en même temps que le cocktail d'élicitation. Par exemple on peut remplacer 1/5^{ème} de la culture cellulaire par un volume équivalent du milieu de prolifération concentré 5 fois.

On considère que la concentration en éléments du milieu de prolifération est proche ou équivalente à zéro après 14, 15 ou 16 jours de phase de prolifération. En particulier on considère que la concentration en éléments minéraux (plus particulièrement les macroéléments et les microéléments) et carbonés (plus particulièrement les sources de carbone) est proche de ou équivalente à zéro après 14, 15 ou 16 jours de phase de prolifération.

Dans un mode de réalisation, le milieu de prolifération selon l'invention au début de la phase d'élicitation comprendra entre autres :
- au moins un macroélément, notamment choisi parmi NH₄NO₃, KNO₃, CaCl₂.2H₂O, MgSO₄.7H₂O, KH₂PO₄ et un mélange de ceux-ci, par exemple à une concentration de macroéléments totale comprise entre 5000 et 8000 mg/L, préférentiellement supérieure à 6000 mg/L de milieu de prolifération, avantageusement entre 6000 et 8000 mg/L ;
- au moins un microélément, notamment choisi parmi KI, H₃BO₃, MnSO₄.4H₂O, ZnSO₄.H₂O, Na₂MoO₄.2H₂O, CuSO₄.5H₂O, CoC1₂.6H₂O, FeSO₄.7H₂O, Na₂EDTA.2H₂O et un mélange de ceux-ci, par exemple à une concentration en microéléments totale comprise entre 10 et 200 mg/L, notamment entre 50 et 150 mg/L de milieu prolifération ;
- au moins une vitamine, notamment choisie parmi le myo-inositol, l'acide nicotinique, la pyridoxine-HCl, la thiamine-HCl et un mélange de ceux-ci, par exemple à une concentration en vitamines totale pouvant aller de 0,01 à 3 g/L, notamment de 0,05 à 1 g/L de milieu de prolifération ;
- au moins un acide aminé, notamment la glycine, par exemple à une concentration dans le milieu de prolifération comprise entre 3 et 4 mg/L de milieu de prolifération ;
- au moins une source de carbone, notamment du saccharose, par exemple à une concentration totale en source de carbone de 10 à 70 g/L de milieu de prolifération, par exemple à environ 30 g/L ;

Dans un mode de réalisation le milieu de prolifération au début de la phase d'élicitation ne comprendra pas, où comprendra une quantité négligeable, notamment moins de 0,001 g/ml, de cytokinine et d'auxine.

Le milieu de prolifération lors de la phase d'élicitation sera avantageusement stérile et préférentiellement à un pH proche de la neutralité.

Le milieu de prolifération lors de la phase d'élicitation pourra notamment avoir la composition suivante : Macroéléments : NH₄NO₃ à 2,8 g/L, KNO₃ à 3 g/L, CaCl₂.2H₂O à 0,45 g/L, MgSO₄.7H₂O à 74 mg/L, KH₂P0₄ à 34 mg/L ; Microéléments : KI à 0,16 mg/L, H₃BO₃ à 6.2 mg/L, MnSO₄.4H₂O à 18,5 mg/L, ZnSO₄.H₂O à 6,6 mg/L, Na₂MoO₄.2H₂Oà 0,25 mg/L, CuSO₄.5H₂O à 0,025 mg/L, CoCl₂.6H₂O à 0,025 mg/L, FeSO₄.7H₂O à 28 mg/L, Na₂EDTA.2H₂O à 37 mg/L ; Vitamines : myo-inositol à 250 mg/L, acide nicotinique à 1,7 mg/L, pyridoxine-HCl à 1 mg/L, thiamine-HCl à 1 mg/L ; Acide aminé : glycine à 4 mg/L ; Source de carbone : saccharose à 30 g/L.

Par « cocktail d'élicitation » selon l'invention, on entend un cocktail permettant de stopper la division cellulaire. Ce cocktail d'élicitation comprend au moins un éliciteur de type composé monocarboxylique et au moins un éliciteur biotique. Le cocktail d'élicitation est introduit, par exemple, à l'aide de solutions mères concentrées dans le milieu de culture.

Par « éliciteur de type composé monocarboxylique » selon l'invention, on entend plus particulièrement le jasmonate de méthyle (MeJa), u un mélange de celui-ci avec l'acide salicylique et/ou l'acide 5-chloro salicylique, L'éliciteur de type composé monocarboxylique sera notamment ajouté de façon à obtenir une concentration finale comprise entre 0,005 et 0,1 g/L, notamment 0,01 et 0,05 g/L de milieu d'élicitation, notamment de 0,002 et 0,004 g/L de milieu d'élicitation.

Par « éliciteur biotique » selon l'invention, on entend plus particulièrement la chitine. La chitine est un polymère linéaire de motif répétitif : béta-1,4 N-acétyl D-glucosamine. L'éliciteur biotique sera notamment ajouté de façon à obtenir une concentration finale comprise entre 0,05 et 50 g/L de milieu d'élicitation, par exemple de 0,1 à 10 g/L, par exemple de 0,5 à 7 g/L, notamment de 1 à 5 g/L de milieu de d'élicitation.

Dans un mode de réalisation, le cocktail d'élicitation comprend du jasmonate de méthyle, notamment à une concentration finale dans le milieu d'élicitation entre 0,002 et 0,005 g/L, et de la chitine, notamment à une concentration finale entre 1 et 4 g/L de milieu d'élicitation.

Dans un mode de réalisation, le cocktail d'élicitation selon l'invention comprendra en outre, au moins un facteur de différenciation cellulaire des cellules végétales et/ou au moins un précurseur de la voie de synthèse des terpènes.

Le « facteur de différenciation cellulaire des cellules végétales » selon l'invention pourra notamment être choisi dans le groupe comprenant, de préférence constitué par, une cytokinine, notamment la benzyl-aminopurine (BAP), l'acide abscissique, la kinétine, le thidiazuron, la 6-γ,γ-diméthylallylaminopurine (2iP ou isopentényladénine) ou la zéatine, une gibbérelline et un mélange de celles-ci, notamment la BAP et/ou la 2iP, en particulier la 2iP.

Le « précurseur de synthèse des terpènes » selon l'invention pourra notamment être choisi dans le groupe comprenant, de préférence constitué par, le pyruvate de sodium ; le pyrophosphate de potassium ; l'acide mévalonique ; le géraniol ; le farnésol ; l'isopentényle, le diméthylallyle, y compris leurs formes pyrophosphatées ; l'acétate de sodium ; l'acide pyruvique et leurs mélanges, notamment le géraniol, le farnésol, le pyruvate de sodium, le pyrophosphate de potassium et leurs mélanges, tel que le pyruvate de sodium et/ou le pyrophosphate de potassium.

La BAP pourra notamment être utilisée à une concentration finale dans le milieu d'élicitation comprise entre 0,01 et 5 mg/L, par exemple entre 0,5 et 5 mg/L de milieu d'élicitation.

L'acide 5-chloro salicylique (5-Chloro SA) pourra notamment être utilisé à une concentration finale dans le milieu d'élicitation comprise entre 0,1 et 15 mg/L.

L'acide salicylique pourra notamment être utilisé à une concentration finale dans le milieu d'élicitation comprise entre 0,1 et 100 mg/L, par exemple entre 20 et 60 mg/L, par exemple environ 45 mg/L.

Le farnésol pourra notamment être utilisé à une concentration finale dans le milieu d'élicitation de 1 à 100 mg/L, par exemple de 15 à 30 mg/L, par exemple à environ 30 mg/L.

Le géraniol pourra notamment être utilisé à une concentration finale dans le milieu d'élicitation de 1 à 100 mg/L, par exemple de 20 à 30 mg/L.

Le pyruvate de sodium pourra notamment être utilisé à une concentration finale dans le milieu d'élicitation de 100 à 5000 mg/L, par exemple de 500 à 2000 mg/L.

Le pyrophosphate de potassium pourra notamment être utilisé à une concentration finale dans le milieu d'élicitation de 1 à 2000 mg/L, par exemple de 100 à 1000 mg/L de milieu d'élicitation.

La 2iP pourra notamment être utilisée à une concentration finale dans le milieu d'élicitation de 0,005 à 10mg/L, par exemple de 0,01 mg/L à 3 mg/L, par exemple de 0,1 à 2 mg/L.

Dans un mode de réalisation selon l'invention, le cocktail d'élicitation comprend du jasmonate de méthyle, de la chitine, du pyruvate de sodium, du pyrophosphate de potassium ou un mélange de ceux-ci.

Dans un mode de réalisation selon l'invention, le cocktail d'élicitation comprend du jasmonate de méthyle, de la chitine, du pyruvate de sodium, du pyrophosphate de potassium, et éventuellement de la BAP et/ou de la 2iP.

Dans un mode de réalisation selon l'invention, le cocktail d'élicitation comprend ou consiste en (les concentrations données entre parenthèses correspondent à la concentration dans le milieu d'élicitation, le cocktail initial pouvant être plus ou moins concentré en fonction de la dilution envisagée) du pyruvate de sodium (de 500 à 2000 mg/L), du pyrophosphate de potassium (de 100 à 1000 mg/L), de la 2iP (de 0,1 à 2 mg/L), du jasmonate de méthyle (de 0,002 à 0,005 g/L) et de la chitine (de 1 à 4 g/L).

Dans un autre mode de réalisation, le cocktail d'élicitation comprend ou consiste en (les concentrations données entre parenthèses correspondent à la concentration dans le milieu d'élicitation, le cocktail initial pouvant être plus ou moins concentré en fonction de la dilution envisagée) de la benzyl-aminopurine (BAP) (de 0,5 à 5 mg/L, notamment de 0,5 à 3 mg/L) ; de l'acide 5-chloro salicylique (5-Chloro SA) (de 2 à 6 mg/L, notamment de 3 à 5 mg/L, par exemple à environ 3 ou à environ 5 mg/L), de l'acide acétyl-salicylique (ASA) et/ou de l'acide salicylique (SA) (de 20 à 60 mg/L, notamment de 30 à 50 mg/L, notamment de 33 à 45 mg/L) ; du jasmonate de méthyle (MeJA) (de 0,002 à 0,005 g/L, notamment de 10 à 40 mg/L) ; de la chitine (de 1 à 4 g/L) ; et du farnésol (F-OH) (de 19 à 40 mg/L) et/ou du géraniol (de 20 à 30 mg/L).

Dans un autre mode de réalisation le cocktail d'élicitation comprend ou consiste en (les concentrations données entre parenthèses correspondent à la concentration dans le milieu d'élicitation, le cocktail initial pouvant être plus ou moins concentré en fonction de la dilution envisagée) du pyruvate de sodium (de 500 à 2000 mg/L), du pyrophosphate de potassium (de 100 à 1000 mg/L), de la 2iP (de 0,1 à 1 mg/L), du jasmonate de méthyle (MeJA) (de 0,002 à 0,005 g/L, notamment de 10 à 40 mg/L) et de la chitine (de 1 à 4 g/L).

Dans un autre mode de réalisation le cocktail d'élicitation comprend ou consiste en (les concentrations données entre parenthèses correspondent à la concentration dans le milieu d'élicitation, le cocktail pouvant être plus ou moins concentré en fonction de la dilution envisagée) du pyruvate de sodium (environ 1,5 g/L), du pyrophosphate de potassium (environ 0,4 g/L), du 2iP (environ 0,4 mg/L), du jasmonate de méthyle (MeJA) (environ 0,03 mg/L), de la chitine (environ 2 g/L).

Pendant la phase d'élicitation, après ajout du cocktail d'élicitation, la culture est maintenue sous agitation, notamment entre 50 et 200 rpm, notamment à environ 125 rpm, pendant une période comprise entre 3 et 30 jours, notamment entre 10 et 25 jours, notamment de 12 à 15 jours, en particulier à une température comprise entre 20 et 35°C, notamment à environ 27°C et avantageusement avec un taux d'oxygène dissout dans le milieu de culture de 2 à 40 %, préférentiellement à environ 16 %. Un apport en air stérile suffisamment enrichi en oxygène pourra être fourni si nécessaire notamment dans le volume mort du bioréacteur ou par diffusion dans le milieu. De préférence, la phase d'élicitation (iii) est menée à l'obscurité. Lors de la phase d'élicitation, la culture cellulaire n'est préférentiellement pas repiquée.

### Etape (iii) - phase de préparation de l'extrait brut enrichi en tripterpènes pentacycliques

Après la phase d'élicitation, le procédé selon l'invention comprend une étape de préparation d'un extrait brut enrichi en tripterpènes pentacycliques, notamment le Célastrol.

L'extrait brut enrichi pourra notamment être obtenu par séparation de la biomasse et du surnageant de culture. La séparation pourra notamment être faite par filtration directe (0-50 µm), par centrifugation ou par décantation des cellules.

Dans un mode de réalisation, l'extrait brut enrichi selon l'invention pourra consister en le surnageant de culture ainsi récupérée.

L'extrait brut enrichi selon l'invention pourra également être obtenu après la lyse de la biomasse. Par exemple, les cellules contenues dans la biomasse récupérée pourront être lysées par une méthode physique (sonication ou broyage) ou chimique (lyse acide) puis ce lysat sera soumis à une extraction par solvant. La phase organique, comprenant notamment les triterpènes issus du cytosol, est ensuite récupérée, notamment par décantation ou centrifugation. Le solvant sera notamment un solvant de type ester, et plus particulière un acétate d'alkyle, l'alkyle étant plus particulièrement linéaire ou ramifié à 1 à 6 atomes de carbone, notamment l'acétate d'éthyle ou l'acétate d'isopropyle. Le volume de solvant utilisé sera notamment de 2 volumes par poids de biomasse.

Préférentiellement, l'extrait brut enrichi selon l'invention correspondra à la phase organique ainsi récupérée.

Alternativement, l'extrait brut enrichi selon l'invention pourra être obtenu après évaporation du solvant et sa substitution notamment par un support adapté au domaine d'utilisation de l'extrait (cosmétique, pharmaceutique) et notamment les huiles végétales.

L'extrait brut enrichi selon l'invention n'est pas purifié. L'extrait enrichi brut pourra éventuellement être purifié en une étape ultérieure.

Lorsque l'extrait brut enrichi est purifié, pour donner un extrait purifié, le procédé selon l'invention comprend en outre une étape (iv) d'obtention d'un extrait purifié d'un ou plusieurs triterpènes pentacycliques à partir de l'extrait enrichi brut obtenu à l'étape (iii).

L'extrait purifié selon l'invention comprendra alors plus de 90 %, notamment plus de 95 %, notamment plus de 98 % d'un ou plusieurs triterpènes pentacycliques selon l'invention. Dans un mode de réalisation l'extrait purifié selon l'invention comprend plus de 90 %, notamment plus de 95 %, notamment plus de 98 % de Célastrol, notamment 100 % de Célastrol.

La purification de l'extrait enrichi selon l'invention pourra notamment être réalisée par une phase de séparation du ou des triterpènes pentacycliques, notamment le Célastrol, la Tingénine A et la Tingénine B, notamment par fractionnement HPLC (chromatographie liquide à haute performance), en particulier les pics à 426 nm comprenant le Célastrol, la Tingénine A et la Tingénine B sortent respectivement à 10,5 min, 8,2 min et 6,5 min. La purification du Célastrol pourra notamment être effectuée comme décrit dans les exemples de la présente demande.

Un autre objet de la description mais pas de l'invention concerne une composition pharmaceutique, telle que dermatologique, ou dermocosmétique comprenant un extrait enrichi brut susceptible d'être obtenu par le procédé selon l'invention et un ou plusieurs excipients pharmaceutiquement ou dermocosmétiquement acceptables.

Les excipients pharmaceutiquement ou dermocosmétiquement acceptables peuvent être tout excipient parmi ceux connus de l'homme de l'art. La composition sera notamment une composition topique, notamment sous forme de crème, d'une lotion, d'un gel, d'une pommade, d'une émulsion, d'une microémulsion, d'un spray, etc.

La composition pharmaceutique, telle que dermatologique, ou dermocosmétique peut en particulier contenir des additifs et aides à la formulation, tels que des émulsionnants, des épaississants, des gélifiants, des fixateurs d'eau, des agents d'étalement, des stabilisants, des colorants, des parfums et des conservateurs.

Un autre objet décrit ici est un extrait enrichi brut obtenu par le procédé selon l'invention, pour son utilisation en tant que médicament, notamment dans le traitement ou la prévention d'une dermatose inflammatoire induisant une réponse cellulaire médiée par TH17.

L'homme du métier pourra facilement déterminer les dermatoses inflammatoires induisant une réponse cellulaire médiée par TH17. L'homme du métier pourra notamment mesurer le niveau d'expression d'IL-22, d'IL-17 et/ou de TNF-α d'une zone lésée par rapport à un échantillon témoin.

La dermatose inflammatoire pourra notamment être sélectionnée dans le groupe comprenant, de préférence constitué par, le psoriasis, la dermatite atopique et l'acné.

La présente invention est illustrée par les figures et exemples non limitatifs détaillés ci-après.

### FIGURES :

FIGURE 1 : Courbe de croissance (trait continu) d'une culture de cellule de *Trypterigium wilfordii* en g/L de biomasse humide (FW) en fonction du temps en jours et concentration en Célastrol (trait en pointillé) en mg de Célastrol par L de suspension cellulaire en fonction du temps en jours.
FIGURE 2 : Chromatogramme HPLC (λ =426 nm) de l'extrait brut enrichi de *Tripterygium wilfordii* obtenu à J33.
FIGURE 3 : Pourcentage d'induction de la synthèse d'IL-17A par des lymphocytes T CD4+ après incubation avec les échantillons 2, 3, 4 et 5 par rapport au témoin négatif incubé avec l'échantillon 1 suivie d'une stimulation avec des anticorps anti-CD3 et anti-CD28 (*** valeur p<0,001).
FIGURE 4 : Pourcentage d'induction de la synthèse d'INF-γ par des lymphocytes T CD4+ après incubation avec les échantillons 2, 3, 4 et 5 par rapport au témoin négatif incubé avec l'échantillon 1 suivie d'une stimulation avec des anticorps anti-CD3 et anti-CD28 (*** valeur p<0,001).
FIGURE 5 : Pourcentage d'induction de la synthèse d'IL-22 par des lymphocytes T CD4+ après incubation avec les échantillons 2, 3, 4 et 5 par rapport au témoin négatif incubé avec l'échantillon 1 suivie d'une stimulation avec des anticorps anti-CD3 et anti-CD28 (*** valeur p<0,001).
FIGURE 6 : Pourcentage d'induction de la synthèse de TNF-α par des lymphocytes T CD4+ après incubation avec les échantillons 2, 3, 4 et 5 par rapport au témoin négatif incubé avec l'échantillon 1 suivie d'une stimulation avec des anticorps anti-CD3 et anti-CD28 (*** valeur p<0,001).
FIGURE 7 : Pourcentage d'induction de la synthèse d'IL-6 par des lymphocytes T CD4+ après incubation avec les échantillons 2, 3, 4 et 5 par rapport au témoin négatif incubé avec l'échantillon 1 suivie d'une stimulation avec des anticorps anti-CD3 et anti-CD28 (*** valeur p<0,001).

### EXEMPLES :

### Exemple 1 : Protocole de dédifférenciation cellulaire

Les cals sont obtenues à partir d'explants de feuilles de *Tripterygium wilfordii.*

Les explants sont stérilisés à l'éthanol à 70 % puis à l'hypochlorite de sodium à 2,5 % de chlore actif, puis rincés à l'eau déminéralisée stérile. Optionnellement, un lavage avec du peroxyde d'hydrogène à 7 % avant le rinçage par l'eau déminéralisée stérile est possible.

Les feuilles sont découpées en morceaux, par exemple en carrés d'environ 8-10 mm de côté. Les explants foliaires sont déposés sur un milieu gélosé de prolifération.

La composition du milieu de prolifération est la suivante :
Macroéléments : NH₄NO₃ à 1650 mg/L, KNO₃ à 1900 mg/L, CaCl₂.2H₂O à 440 mg/L, MgSO₄.7H₂O à 370 mg/L, KH₂PO₄ à 170 mg/L,
Microéléments : Kl à 0,83 mg/L, H₃BO₃ à 6,2 mg/L, MnSO₄.4H₂O à 22,3 mg/L, ZnSO₄.H₂O à 6,61 mg/L, Na₂MoO₄.2H₂Oà 0,25 mg/L, CuSO₄.5H₂O à 0,025 mg/L, CoCl₂.6H₂O à 0,025 mg/L, FeSO₄.7H₂O à 27,8 mg/L, Na₂EDTA.2H₂O à 37,3 mg/L,
Vitamines: myo-inositol à 100 mg/L, acide nicotinique à 0,5 mg/L, pyridoxine-HCl à 0,5 mg/L, thiamine-HCl à 0,5 mg/L,
Acide aminé : glycine à 2 g/L,
Source de carbone : saccharose à 30 g/L,
Hormones végétales : kinétine à 0,1 mg/L, acide 2,4-dichloro-phénoxyacétique (2,4D) à 0,5 mg/L, acide naphthalène acétique (NAA) à 1 mg/L.

Le milieu de prolifération est gélifié par addition d'agar à 8-12 g/L, son pH est ajusté à pH 6 ± 0,5 (avec du KOH, 1M) avant un autoclavage de 20 min à 121°C. Les boîtes de pétri contenant les explants sont incubées à l'obscurité à 27-28°C.

Le repiquage des cals est réalisé tous les mois sur le même milieu gélosé. Pour cela les cals obtenues sont détachées de l'explant foliaire et déposées sur de nouvelles géloses de milieu de prolifération.

### Exemple 2 : Formulation des milieux de culture et de propagation (phase de prolifération)

Après quelques mois de repiquage, des cals friables sont obtenues et sont transférées dans du milieu de prolifération liquide.

Le milieu de prolifération a, par exemple, la composition indiquée ci-dessous :
Macroéléments : NH₄NO₃ à 1650 mg/L, KNO₃ à 2500 mg/L, CaCl₂.2H₂O à 440 mg/L, MgSO₄.7H₂O à 370 mg/L, KH₂PO₄ à 130 mg/L,
Microéléments : KI à 0,41 mg/L, H₃BO₃ à 6,2 mg/L, MnSO₄.4H₂O à 22,3 mg/L, ZnSO₄.H₂O à 7,5 mg/L, Na₂MoO₄.2H₂Oà 0,25 mg/L, CuSO₄.5H₂O à 0,025 mg/L, CoCl₂.6H₂O à 0,025 mg/L, FeSO₄.7H₂O à 19,85 mg/L, Na₂EDTA.2H₂O à 26,64 mg/L,
Vitamines : myo-inositol à 50 mg/L, acide nicotinique à 0,25 mg/L, pyridoxine-HCl à 0,25 mg/L, thiamine-HCl à 0,25 mg/L,
Sources de carbone : saccharose à 30 g/L,
Hormones végétales : kinétine à 0,083 mg/L, acide 2,4-dichloro-phénoxyacétique (2,4D) à 0,575 mg/L, acide naphthalène acétique (NAA) à 0,350 mg/L.

Le pH du milieu est ajusté à pH 6 ± 0,5 (par ajout de KOH, 1M) avant un traitement de stérilisation approprié, par exemple autoclave à 121°C pour une durée minimale de 20 minutes ou par filtration stérilisante sur 0,2 µm.

La mise en suspension cellulaire est réalisée en déposant environ 40 g de cals friables dans un Erlenmeyer de 200 ml contenant le milieu de propagation, incubation pendant une semaine sur une table d'agitation à 100 RPM (rotation par minute) à l'obscurité à 27-28°C. Le surnageant cellulaire est récolté à la pipette laissant les amas de cals résiduels. La suspension cellulaire obtenue est cultivée pendant 15 jours puis propagée par dilution au 1/5^{ème} dans du nouveau milieu tous les 15 jours.

Le taux de remplissage des Erlenmeyers est de 40 % (80 mL) et le taux d'inoculation par transfert de suspension cellulaire est de 20-25 % du volume, soit environ 160 g/L de biomasse fraîche. La culture est menée ainsi pendant 15 jours à l'obscurité à 27-28°C sous agitation orbitale de 110-120 RPM. A ce stade la biomasse est présente à une concentration de 300 g/L environ de biomasse fraîche par litre de suspension.

### Exemple 3: Production de Triterpènes en Erlenmeyer (phase d'élicitation et de préparation de l'extrait brut enrichi).

### Phase d'élicitation :

Après les 15 jours de culture, 1/5^{ème} de la culture cellulaire est retirée de l'Erlenmeyer et 20 mL d'un milieu de prolifération concentré est ajouté dans l'Erlenmeyer. La composition du milieu concentré est la suivante : Macroéléments : NH₄NO₃ à 13,9 g/L, KNO₃ à 15,2 g/L, CaCl₂.2H₂O à 2,2 g/L, MgSO₄.7H₂O à 370 mg/L, KH₂PO₄ à 170 mg/L ; Microéléments : KI à 0,83 mg/L, H₃BO₃ à 31,2 mg/L, MnSO₄.4H₂O à 91,5 mg/L, ZnSO₄.H₂O à 33,05 mg/L, Na₂MoO₄.2H₂Oà 1,25 mg/L, CuSO₄.5H₂O à 0,125 mg/L, CoCl₂.6H₂O à 0,125 mg/L, FeSO₄.7H₂O à 139 mg/L, Na₂EDTA.2H₂O à 186,5 mg/L ; Vitamines : myo-inositol à 1250 mg/L, acide nicotinique à 8,5 mg/L, pyridoxine-HCl à 5 mg/L, thiamine-HCl à 5 mg/L ; Acide aminé : glycine à 20 mg/L ; Source de carbone : saccharose à 150 g/L (solubilisé dans de l'eau déminéralisée).

Le cocktail d'élicitation est ensuite ajouté dans l'Erlenmeyer au milieu de prolifération à l'aide de solutions mères réalisées dans du diméthylsulfoxyde. La composition du cocktail éliciteur permet d'obtenir les concentrations suivantes dans le milieu d'élicitation (+ cellules): pyruvate de sodium 1,5 g/L, pyrophosphate de potassium 0,440 g/L, 2iP 0,0004 g/L, jasmonate de méthyle 0,036 g/L et chitine 2 g/L.

La production de Célastrol et ses dérivés est menée pendant 12 jours à l'obscurité à 27-28°C sous agitation orbitale de 120 rpm.

### Récolte de la biomasse pour extraction :

A l'arrêt de la culture, on filtre le milieu pour récupérer la totalité de la biomasse qui contient la majorité de Célastrol.

Après séparation de la biomasse par filtration 0-50 µm, 2 volumes de solvant de type ester et plus particulièrement acétate d'alkyle, notamment l'acétate d'éthyle (on encore l'acétate d'isopropyle), sont mélangés à 1 poids de biomasse. Ce mélange est soumis à une extraction par sonication permettant de lyser les cellules et rendre disponible les éléments du cytosol. La phase organique comprenant la fraction triterpénique est récupérée après centrifugation du mélange.

La concentration en Célastrol dans la phase organique est mesurée. La concentration en Célastrol par litre de suspension est estimée à 553 mg, ce qui correspond à un poids de 0,0166 g de Célastrol par gramme en poids sec de cellules.

### Exemple 4 : Production de Triterpènes (Célastrol et dérivés) en bioréacteurs en poches à usage unique de type WAVE

L'exemple illustré est décrit pour des réacteurs de type WAVE, par exemple, de marque Sartorius, pour des volumes de 5 L ou 10 L ou 2 X 5 L, mais la méthode peut être adaptée et appliquée à des volumes supérieurs et à des matériels issus d'autres fabricants.

Le système binaire décrit précédemment pour des bioréacteurs traditionnels de laboratoire en verre ou industriel en inox est appliqué de la même manière avec les 2 poches WAVE.

### Prolifération :

Le bioréacteur Wave A (5 L), placé sur son support, est rempli du milieu de prolifération par filtration stérilisante en ligne et gonflé par de l'air.

Une pré-culture de *Trypterygium wilforddi* est réalisée pendant 15 jours en Erlenmeyer comme décrit à l'exemple 2. Le milieu de prolifération du bioréacteur est ensuite ensemencé par cette pré-culture à une concentration de 160 g/L (poche A).

Le bioréacteur est incubé selon les conditions suivantes :
- angle de basculement : 5-8° ;
- fréquence de basculement : 16-30 RPM ;
- débit d'aération : 0,1-0,5 L/mn d'air enrichi à 50 % en oxygène pur ;
- température : 27°C ;
- durée : 17 jours.

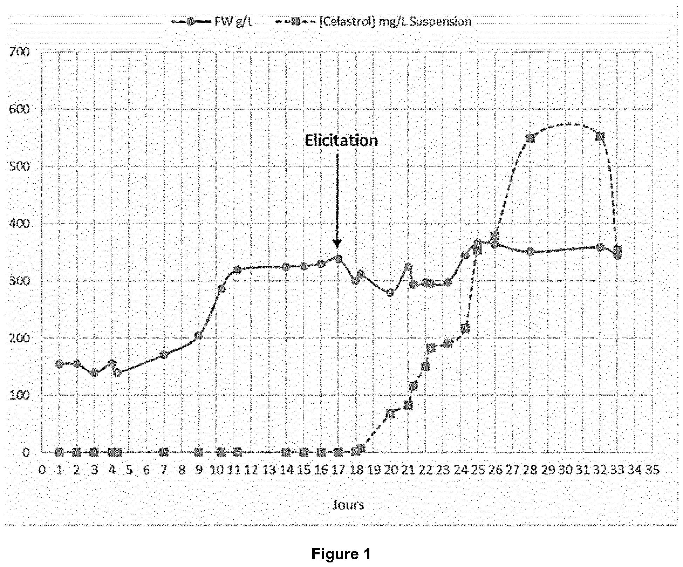

Lors de cette phase de prolifération la croissance des cellules est mesurée tous les jours (Figure 1).

### Elicitation et Production de Triterpènes (Célastrol et dérivés) :

Un volume d'environ 1000 ml de culture de la poche A (5 L) est transféré dans la poche B placée à côté de la poche A sur le même plateau. Le milieu de la poche A est complémenté par 1000 mL de milieu concentré dans de l'eau déminéralisée ayant la composition suivante : Macroéléments :NH₄NO₃ à 13,9 g/L, KNO₃ à 15,2 g/L, CaCl₂.2H₂O à 2,2 g/L, MgSO₄.7H₂O à 370 mg/L, KH₂PO₄ à 170 mg/L; Microéléments: KI à 0,83 mg/L, H₃BO₃ à 31,2 mg/L, MnSO₄.4H₂O à 91,5 mg/L, ZnSO₄.H₂O à 33,05 mg/L, Na₂MoO₄.2H₂Oà 1,25 mg/L, CuSO₄.5H₂O à 0,125 mg/L, CoCl₂.6H₂O à 0,125 mg/L, FeSO₄.7H₂O à 139 mg/L, Na₂EDTA.2H₂O à 186,5 mg/L; Vitamines: myo-inositol à 1250 mg/L, acide nicotinique à 8,5 mg/L, pyridoxine-HCl à 5 mg/L, thiamine-HCl à 5 mg/L ; Acide aminé : glycine à 20 mg/L ; Source de carbone : saccharose à 150 g/L ; auquel on ajoute le cocktail d'élicitation suivant : pyruvate de sodium 7,5 g/L, pyrophosphate de potassium 2,2 g/L, 2iP 0,002 g/L, jasmonate de méthyle 1,8 g/L et chitine 10 g/L.

Le contenu de la poche A est ainsi élicité sous agitation à une température de 27°C.

La culture en phase d'élicitation est suivie par la mesure de la croissance cellulaire et de la concentration en Célastrol dans la culture pendant 16 jours (Figure 1).

On peut constater que la concentration de Célastrol dans la poche A augmente régulièrement jusqu'à J32 et est à son maximum à 553 mg par litre de milieu d'élicitation après 15 jours d'incubation.

La vitesse de production du Célastrol est d'environ 46 mg/L de suspension cellulaire et par jour durant les 15 jours après élicitation et ce jusqu'à J32.

La cinétique de production de Célastrol commence à s'infléchir peu de temps après avoir consommé la quasi-totalité du saccharose disponible (Figure 1).

### Exemple 5: Production d'un extrait enrichi en Célastrol par extraction solide/liquide de la biomasse issue de suspension cellulaire de Tripterygium wilfordii (TW08)

A 15 jours après élicitation (32 jours après inoculation), la majorité de la biomasse est récupérée par filtration de la suspension cellulaire avec un filtre nylon (20 - 50 µM) (TW08).

A partir de 5 L de suspension, on récupère environ 1925 g de biomasse. Cette biomasse est extraite avec de l'acétate d'éthyle (on encore de l'acétate d'isopropyle) en proportion 2 : 1 (Vol : Poids) par rapport au poids de biomasse (ici 3850 mL de solvant pour 1925 g de biomasse). Le mélange biomasse/solvant est alors soumis à une extraction physique, par sonication. La phase organique est alors récupérée après macération sous agitation. L'ajout de solvant (suivi d'une macération sous agitation et la récupération de la phase organique) est répété 2 fois.

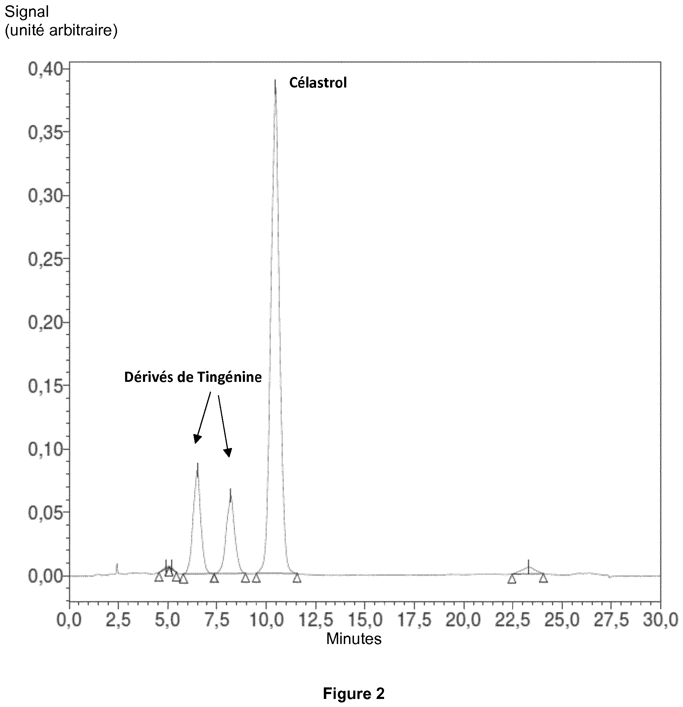

La concentration en Célastrol et dérivés dans la phase organique est mesurée par dosage HPLC (quantification Célastrol, dérivés de Tingénine) (voir Figure 2) Conditions expérimentales : Colonne Waters Atlantis dC18 4,6 x 150 mm - 5µ équipée d'une colonne de garde Atlantis dC18 5 µm 4,6 x 20 mm Gd Column - 5µ. Phases mobiles : (A) Acétate d'ammonium 0,1 mM pH 4,0 (B) Acétate d'ammonium 0,1 mM pH 4,0 dans acétonitrile 99,8 %. Gradient: initial 25 %(A)/75 %(B) - 20 min 0 %(A)/100 %(B) - 24,5 min 0 %(A)/100 %(B) - 25 min 25 %(A)/75 %(B) - 30 min 25 %(A)/75 %(B). Débit : 1 mL/mn. Détection à 426 nm : pics de rétention (min) : Tingénine B (6,5), Tingénine A (8,2), Célastrol (10,5).

### Exemple 6: Purification du Célastrol à partir de la culture de Tripterygium wilfordii

A partir de l'extrait obtenu à l'exemple 5, l'acétate d'éthyle (ou l'acétate d'isopropyle) est évaporé sous pression réduite afin d'obtenir un extrait sec. Une partie de l'extrait ainsi obtenu est purifié dans un premier temps par chromatographie liquide moyenne pression (CLMP) sur silice (40 g, 125 x 25 mm, 30 µm) avec un gradient d'élution CH₂Cl₂/MeOH (100/0 à 0/100). Toutes les fractions obtenues sont analysées par Chromatographie sur Couche Mince (CCM - Phase stationnaire : Silice 60 Å ; Phase mobile : toluène / acétate d'éthyle / acide acétique 70 : 33 : 3) et les fractions contenant majoritairement le Célastrol et ses dérivés Tingénine (A et B) sont rassemblées. Dans un deuxième temps, la fraction contenant les produits d'intérêts (Celastrol et dérivés) est purifiée par chromatographie liquide haute performance (HPLC) sur phase inverse (Lichrospher 100RP18, 250 x 25 mm, 5 µM) avec un gradient linéaire Eau/Acétonitrile/0,1 % acide formique (80/20 à 0/100). Le pic du Célastrol est collecté et concentré sous pression réduite, on obtient environ 2,5 à 3 g de Célastrol à partir de 5 L de suspension de culture de départ. Eventuellement, il peut être cristallisé pour avoir une pureté absolue.

En parallèle, une partie de l'extrait sec obtenu est repris dans un tampon pour donner l'extrait R003034 qui sera évalué en activité pharmacologique anti-Th17.

### Exemple 7 : Activité anti-Th17 sur cellules CD4+ humaines de l'extrait CCV enrichi en Célastrol.

Dans les dermatoses inflammatoires (notamment dans le psoriasis), il est connu que les cellules T CD4+ surexpriment les interleukines IL17, IL6 et IL22, ainsi que l'IFN gamma et le TNF alpha. Dans le cas de la dermatite atopique, IL17 est surexprimé dans une certaine phase de la pathologie. En outre, la voie Th17 est fortement activée en cas d'acné. Le pouvoir inhibiteur d'un extrait enrichi selon l'invention sur la surexpression de ces molécules a été testé.

Les cellules CD4+ humaines ont été isolées à partir des cellules mononucléées du sang de 2 donneurs sur Ficoll Paque plus® selon le protocole préconisé par le fabricant (GE Healthcare). Les lymphocytes CD4+ sont isolés par tri positif par le kit Miltenyi Biotec (CD4) et une colonne LS column et sont remis en suspension dans un milieu de culture RPMI (Sigma-Aldrich : contenant de la L-glutamine et 10% de sérum fœtal bovin) complété avec 100 µg/ml de streptomycine et 100 U/ml de pénicilline. Les lymphocytes en suspension sont répartis dans des puits de microplaques. Différents extraits ou contrôles sont ensuite ajoutés dans les puits :
1. contrôle négatif (le même volume de tampon sans réactif est ajouté) ;
2. extrait R003034 à 0,06 mg/ml (titré en Célastrol à 9 ng/ml) (concentration finale dans le puits) ;
3. extrait R003034 à 0,2 mg/ml (titré en Célastrol à 30 ng/ml) (concentration finale dans le puits) ;
4. extrait R003034 à 0,6 mg/ml (titré en Célastrol à 90 ng/ml) (concentration finale dans le puits) ;
5. dexaméthasone à 2 µM (contrôle positif) (concentration finale dans le puits).

Après 2 heures d'incubation à température ambiante, les lymphocytes CD4+ sont activés à 37°C pendant 20 heures avec des anticorps anti-CD3 et anti-CD28 à une concentration finale respective de 300 ng/ml et de 400 ng/ml. Les anticorps anti-CD3 et anti-CD28 sont connus pour induire une réponse de type Th17.

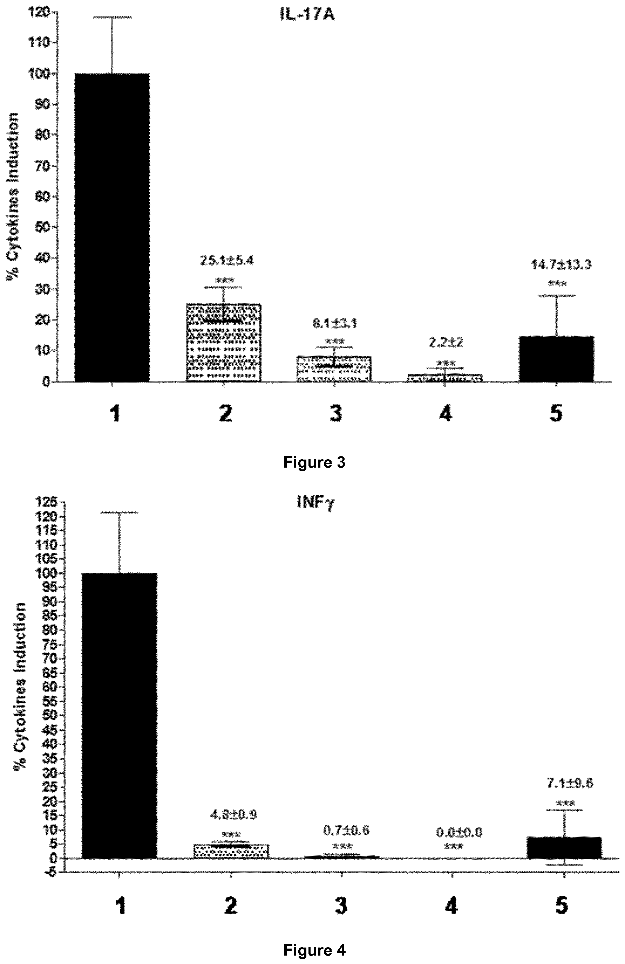

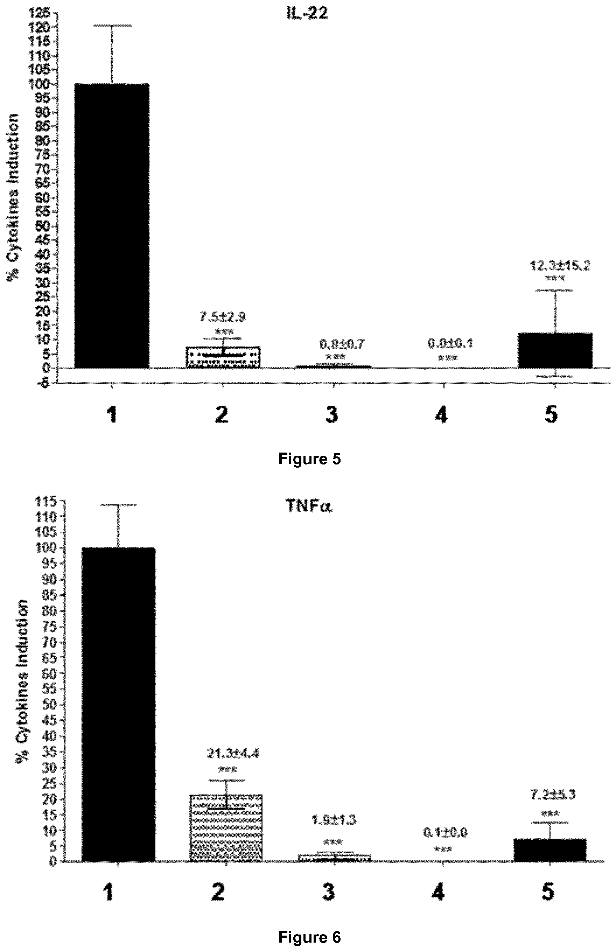

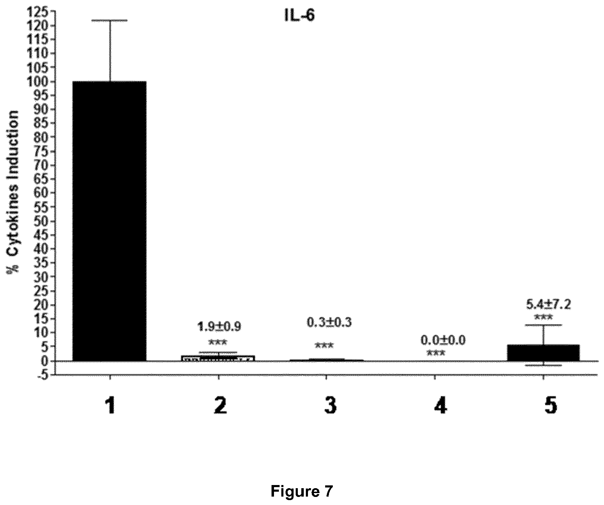

Les cytokines IL-17A, INF gamma, IL-22, TNF alpha et IL-6 ont été quantifiées dans le surnageant de chaque tube par la méthode de Multiplex Immunoassays (Jager *et al.* 2003). Les résultats obtenus sont issus de 2 expériences avec des lymphocytes provenant de 2 donneurs différents. Les résultats sont présentés en Figures 3 à 7 en pourcentage d'induction par rapport au témoin négatif (1). On constate que :
- le contrôle positif 5 (le dexaméthasone (2 µM)) inhibe la surexpression de toutes les cytokines : IL-17A, INF gamma, IL-22, TNF alpha et IL-6 ;
- l'extrait selon l'invention (2, 3 et 4) inhibe fortement la surexpression des cytokines IL-17A, INF gamma, IL-22, TNF alpha et IL-6. L'inhibition de la surexpression d'IL-17A et TNF alpha est dose-dépendante. Dès la plus faible dose, titrée à 9 ng/ml en Célastrol, on constate une inhibition de la surexpression de l'IL-17A de 75 %, une inhibition de la surexpression du TNF alpha de 80 % et une inhibition presque totale de la surexpression de l'INF-gamma, de l'IL-22 et de l'IL-6.

Ceci démontre que l'extrait R003034 a une activité anti-Th17 très forte, comparable sinon supérieure à la dexaméthasone (2 µM), ce qui lui confère une activité remarquable dans le traitement de pathologies Th17-dépendantes comme le psoriasis, l'acné et la dermatite atopique.

### Exemple 8 : Comparaison des rendements en célastrol obtenus à partir de cultures de cellules végétales de Tripterygium wilfordii.

Dix cultures en erlenmeyer (volume = 50 mL) ont été réalisées en parallèle, à partir d'une même lignée cellulaire de *Tripterygium wilfordii,* en duplicata dans les mêmes conditions d'agitation, d'aération et de température. Dès la fin de la phase de croissance, la densité de la suspension étant maximale, *la quantité de biomasse fraîche pesée était équivalente* (poids en moyenne de 340 g de biomasse fraîche/L) dans chacun des erlenmeyers. A cet instant, les éliciteurs ont été ajoutés dans le milieu de culture des dix erlenmeyers aux concentrations indiquées dans le tableau ci-dessous, dans lequel le rendement est exprimé en mg de Célastrol/kg de biomasse fraîche.

| | *Exemples de référence* | | | *Exemples impliquant l'utilisation d'un cocktail de 2 éliciteurs* | | | | | | |
|---|---|---|---|---|---|---|---|---|---|---|
| Erlen | E1 | E2 | E3 | E4 | E5 | E6 | E7 | E8 | E9 | E10 |
| MeJa (µM) | 0 | 0 | 64 | 36 | 36 | 64 | 64 | 64 | 92 | 92 |
| Chitine (g/L) | 0 | 2 | 0 | 2 | 4,2 | 1,3 | 2,5 | 3,7 | 3,1 | 4,2 |
| Rendement | 4 ,5 | 55 | 738 | 1730 | 2178 | 1506 | 1724 | 2025 | 1986 | 1878 |

Le dosage du Célastrol en suspension a été réalisé par HPLC selon l'exemple 5, une courbe d'étalonnage ayant été réalisée avec des produits témoins.

Les suspensions contenues dans les erlenmeyers notés E1, E2, E3, ont été élicitées avec une solution témoin ne comportant aucun éliciteur pour E1, avec la chitine seule (2 g/L) pour E2 et avec le MeJa seul (64 µM) pour E3. La concentration en MeJa dans E3 est équivalente à celle décrite par Liu *et al.* 2016, c'est-à-dire 64µM.

Les suspensions contenues dans les erlenmeyers notés E4 à E10 ont été élicitées à trois concentrations différentes en MeJa (36, 64 et 92µM) et cinq concentrations différentes en Chitine (1,3 ; 2 ; 2,5 ; 3,1 et 4,2 g/L).

Il est constaté que les rendements en Célastrol obtenus pour E6, E7 et E8 sont augmentés respectivement de 204 %, 233 % et 274 % par rapport à E3, la concentration en MeJa étant la même dans ces quatre erlens. Un rendement encore meilleur est observé pour E5, dans lequel la concentration en MeJa est différente.

### Exemple 9 : Activité inhibitrice vis-à-vis de NFκB

Le facteur de transcription NPκB contrôle l'expression d'un grand nombre de gènes impliqués dans la régulation de la réponse inflammatoire. A l'état inactif, NPκB est séquestré dans le cytoplasme par la protéine IKB. Certains stimuli pro-inflammatoires tels que le TNF alpha et l'IL-1 bêta conduisent à l'activation de NPκB, c'est-à-dire à sa translocation nucléaire. Une fois dans le noyau, NPκB va induire la transcription de gènes pro-inflammatoires codant des cytokines, des chémokines, des molécules d'adhésion, des facteurs de croissance et des enzymes inductibles.

Les activités anti-inflammatoires des extraits obtenus à partir des suspensions contenues dans les erlenmeyers E1, E2, E3 et E6 de l'exemple 8 ont été évaluées *in vitro* sur des kératinocytes humains HaCat selon la méthode décrite par Albanesi *el al.* Les extraits pesés en poids sec ont été repris dans un tampon et introduits dans une solution de culture de cellules HaCat en concentrations finales équivalentes (ng/mL) pour chaque extrait, et ont été incubés pendant 1 h. L'expression de NPκB est ensuite induite par stimulation avec TNF alpha (0,3 ng/mL).

| | TNF alpha | Dex | Extrait issu de E1 | | Extrait issu de E2 | | Extrait issu de E3 | | Extrait issu de E6 | |
|---|---|---|---|---|---|---|---|---|---|---|
| Conc. | 0,3 | 2 µM | 0,19 | 0,58 | 0,19 | 0,58 | 0,19 | 0,58 | 0,19 | 0,58 |
| % inh. | 0 | 42,5 | -1 | -9 | -7 | 2 | 3 | 1 | 20 | 68 |
| sem | 4 | 6 | 2 | 3 | 3 | 3 | 2 | 3 | 3 | 4 |

| | | | | | | | | | | |
|---|---|---|---|---|---|---|---|---|---|---|
| Dex : Dexaméthasone ; Conc. : concentration (ng/mL) ; %inh : % d'inhibition de l'expression de NPκB par rapport à l'induction de TNF alpha ; sem : erreur standard moyenne. | | | | | | | | | | |

La dexaméthasone à 2µM, utilisée comme contrôle positif, inhibe de plus de 40 % l'expression de NFκB.

L'extrait selon la présente invention (obtenu à partir de E6, avec le couple d'éliciteurs MeJa et chitine) évalué aux concentrations de 0,19 et 0,58 ng/mL inhibe respectivement de 20 % et 70 % l'expression de NFκB.

L'extrait préparé selon Liu *et al.* (à partir de E3, avec MeJa comme seul éliciteur) évalué aux mêmes concentrations n'induit aucune inhibition notable de l'expression de NFκB. De même, les extraits issus de E2 (avec la chitine comme unique éliciteur) et du témoin E1 n'ont aucun effet sur l'expression de NFκB.

Seul l'extrait issu de culture élicitée selon la présente invention inhibe fortement NFκB, et de manière concentration-dépendante.

### Exemple 10 : Activité antimicrobienne vis-à-vis de Propionibacterium acnes d'un extrait préparé selon le procédé de la présente invention comparée à celle d'autres composés

L'activité est évaluée sur la souche *Propionibacterium acnes* (CIP 53117T), avec une suspension d'inoculation à 10⁸ UFC/mL. Le milieu d'entretien est composé de gélose Columbia + 5% de sang de mouton, avec une incubation durant 24h à 36 ± 1 °C sous anaérobiose. Le milieu d'essai est composé de bouillon Mueller Hinton + 10 % de sérum de veau fœtal (SVF) et gélose Columbia, + 5 % de sang de mouton, avec une incubation durant 24 h à 36 ± 1 °C sous anaérobiose. La détermination des concentrations minimales inhibitrices (CMI) est réalisée par microméthode en milieu liquide. 100 µL de milieu de culture liquide sont déposés dans chaque puits d'une microplaque stérile 96 puits. 100 µL du produit à tester sont déposés dans le premier puits d'une ligne. Des dilutions de raison 2 sont ensuite réalisées des puits 1 à 10. Les suspensions-test de *Propionibacterium acnes* sont préparées extemporanément dans du Tryptone sel. 100 µL sont déposés dans chaque puits d'une seconde microplaque, à l'exception de la colonne 11. L'ensemble de la première microplaque est inoculé, à l'aide d'un ensemenceur multipoint Denley, à partir de la seconde microplaque. Après incubation, les CMI sont définies comme la plus grande dilution avec absence de croissance visible. Les colonnes 11 et 12 servent respectivement de témoin négatif et positif de croissance. Deux références ont été testées en parallèle. La détermination des concentrations minimales bactéricides (CMB) est réalisée par repiquage des microplaques de CMI sur milieu gélosé, à l'aide de l'ensemenceur multipoint Denley. Après incubation, la CMB est définie comme la plus grande dilution avec absence de croissance visible. Tous les essais sont réalisés en duplicate.

Les composés testés sont :
- le célastrol purifié dans le DMSO, (solution mère = 150 µg/mL / 10 % DMSO)
- l'extrait préparé selon le procédé de la présente invention dans le pentylène glycol, (solution mère = 2 % / 2 % pentylène glycol)
- la Tingénine A dans le DMSO, (solution mère = 150 µg/mL / 10 % DMSO)
- la Tingénine B dans le DMSO, (solution mère = 150 µg/mL / 10 % DMSO)
- les références sont l'amoxicilline, le DMSO (solution mère = 100 %), et le pentylène glycol (solution mère = 2 %)

Les résultats obtenus sont résumés dans le tableau ci-dessous, qui indique les valeurs de CMI et CMB pour les 4 échantillons et les deux excipients. La concentration maximale d'essai correspond à la concentration de la solution mère / 2.

| Composés | CMI (µg/mL) | CMB (µg/mL) |
|---|---|---|
| Célastrol | 0,59 | 1,17 |
| DMSO | 5 | 5 |
| Extrait selon le procédé | 0,25* | 0,25* |
| Pentylène glycol | >1 | >1 |
| Tingénine A | 2,34 | 2,34 |
| Tingénine B | 1,17 | 1,17 |

| | | |
|---|---|---|
| * : soit 0,375 µg/mL en équivalent célastrol. | | |

En conclusion, le célastrol présente une activité antibactérienne sur *Propionibacterium acnes* tout à fait intéressante, mais il apparait que c'est l'extrait préparé selon le procédé de l'invention qui présente le niveau d'activité antibactérienne sur *Propionibacterium acnes* le plus important. La Tingénine B présente une activité antibactérienne supérieure à celle observée avec la Tingénine A.

### Exemple 11 : Activité antimicrobienne vis-à-vis de Staphylococcus aureus d'un extrait préparé selon le procédé de la présente invention comparée à celle d'autres composés

Il est connu que le S. *aureus* est un pathogène qui colonise les lésions des patients atteints de la dermatite atopique, et est également présent chez les patients atteints de psoriasis (Tomi *et al.* 2005).

L'activité antibactérienne contre le *S*. *aureus* de l'extrait obtenu selon le procédé de la présente invention a été évaluée. L'expérience a été réalisée comme décrit dans l'exemple 10, avec la souche pathogénique S. *aureus* CIP 4.83, le milieu d'entretien Gélose Trypticase-Soja, le milieu d'essai CMI/CMB bouillon et gélose Mueller Hinton et une incubation à 36°C pendant 24 h.

Les résultats obtenus sont résumés dans le tableau ci-dessous. On constate que si le Célastrol a une forte activité antibactérienne, l'activité anti-*S*. *aureus* de l'extrait obtenu selon le procédé de la présente invention titré en équivalent en Célastrol est encore meilleure. Par ailleurs, la molécule Tingénine B a une meilleure activité que la Tingénine A.

| Composés | CMI (µg/mL) | CMB (µg/mL) |
|---|---|---|
| Célastrol | 0,29 | 0,59 |
| DMSO | >5 | >5 |
| Extrait selon le procédé | 0,125* | 0,25** |
| Pentylène glycol | >1 | >1 |
| Tingénine A | 1,17 - 2,34 | 2,34 |
| Tingénine B | 0,59 | 1,17 |

| | | |
|---|---|---|
| * 0,1875 µg/mL en équivalent célastrol ; ** 0.375 µg/mL en équivalent célastrol. | | |

En conclusion, cet extrait possède non seulement une activité pharmacologique anti-Th17 intéressante, mais de manière surprenante une forte activité anti-S. *aureus,* ce qui le rend très attrayant comme actif dans le traitement topique de la dermatite atopique et du psoriasis.

### Exemple 12 : Crème pour application topique

| **Produits** | **Pourcentage massique** |
|---|---|
| glycérine | 8 à 10 % |
| copolymère d'hydroxyacrylate | 1,8 à 2 % |
| gomme xanthane | 0,1 à 0,3 % |
| cétéareth 33 et alcool cétéarylique | 4 à 6 % |
| stéarate de glycéryle | 1,5 à 2 % |
| palmitate d'éthylhexyle | 12 à 15 % |
| extrait de *Tripterygium wilfordii* (en solution ;10 à 600 mg/L) | 0,1 à 2,0 % |
| acide glycolique | 2 à 4 % |
| Lactamide MEA | 3 à 6 % |
| Laureth-9 | 1 à 3 % |
| sulfonate d'huile de schiste sodique | 1 à 3 % |
| extrait de racine de chicorée | 1 à 3 % |
| Eau complétée à | 100 % |

L'extrait de *Tripterygium wilfordii* est préparé selon la présente invention, sa concentration étant exprimée en poids d'extrait sec dans un solvant compatible avec la formulation, tel que l'huile d'olive, le pentylène glycol ou le myritol 318 ou autres.

Cet exemple de formulation n'est en aucun cas limitatif, et peut être adapté selon le traitement.

Ainsi, pour le traitement du cuir chevelu, un shampoing pourra être formulé en faisant appel aux connaissances de l'homme de l'art.

### REFERENCES:

Camelio et al. JACS 2015, 137:11864-67
Coppede et al. Plant Cell Tiss. Organ Cuit. 2014, 118:33-43
Jager et al. Clin. Diagn. Lab. Immunol. 2003, 10(1):133-9
Kelhala et al. PLOS One 2014, 9(8):e105238
Liu et al. J. Asian Nat. Prod. Res. 2016, 19:1-10
Lowes et al. Annu. Rev. Immunol. 2014, 32:227
Miyagaki et al. J. Derm. Science 2015, 78:89
Murashige & Skoog Physiol. Plant. 1962, 15: 473-497
Albanesi el al. Curr. Drug Targets Inflamm. Allergy 2005, 4(3):329-334
Tomi et al. J. Am. Acad. Dermatol. 2005, 53(1):67-72.

## Revendications

1. Procédé de production d'un extrait brut enrichi en un triterpène pentacyclique qui est le célastrol comprenant les étapes suivantes :
(i) une phase de prolifération de cellules d'une plante de la famille des Celastraceae qui est *Tripterygium wilfordii* dans un milieu de prolifération,
(ii) une phase d'élicitation par ajout d'un cocktail d'élicitation à la culture cellulaire obtenue à l'étape (i), ledit cocktail d'élicitation comprenant au moins un éliciteur de type composé monocarboxylique qui est le jasmonate de méthyle et au moins un éliciteur biotique qui est la chitine, et
(iii) la préparation d'un extrait brut enrichi en tripterpènes pentacycliques à partir de la culture cellulaire obtenue à l'étape (ii).

2. Procédé de production d'un extrait brut enrichi selon la revendication 1, dans lequel le cocktail d'élicitation comprend en outre au moins un facteur de différenciation cellulaire des cellules végétales et au moins un précurseur de la voie de synthèse des terpènes.

3. Procédé de production d'un extrait brut enrichi selon la revendication 2, dans lequel le au moins un facteur de différenciation cellulaire des cellules végétales est sélectionné parmi une cytokine, une gibbérilline et un mélange de celles-ci.

4. Procédé de production d'un extrait brut enrichi selon la revendication 3, dans lequel le au moins un facteur de différenciation cellulaire des cellules végétales est sélectionné parmi la benzylaminopurine (BAP), la 6-γ,γ-diméthylallylaminopurine (2iP) et un mélange de celles-ci.

5. Procédé de production d'un extrait brut enrichi selon l'une quelconque des revendications 2 à 4, dans lequel le au moins un précurseur de synthèse des terpènes est choisi dans le groupe constitué par le pyruvate de sodium, le pyrophosphate de potassium et un mélange de ceux-ci.

6. Procédé de production d'un extrait brut enrichi selon l'une quelconque des revendications 1 à 5, comprenant en outre une étape (iv) d'obtention d'un extrait purifié du triterpène pentacyclique à partir de l'extrait brut enrichi obtenu à l'étape (iii).

## Patentansprüche

1. Verfahren zur Herstellung eines Rohextrakts, das mit einem pentacyclischen Triterpen angereichert ist, das Celastrol ist, das die folgenden Schritte umfasst:
(i) eine Phase der Zellproliferation einer Pflanze aus der Familie der Spindelbaumgewächse (Celastraceae), die Wilfords Dreiflügelfrucht (*Tripterygium wilfordii*) ist, in einem Proliferationsmedium,
(ii) eine Phase der Elizitation durch Beimengung eines Elizitationscocktails zu der im Schritt (i) erhaltenen Zellkultur, wobei der Elizitationscocktail mindestens einen Elizitor vom Typ Monocarbonverbindung, die Methyljasmonat ist, und mindestens einen biotischen Elizitor umfasst, der Chitin ist, und
(iii) Vorbereitung eines Rohextrakts, das mit pentacyclischen Triterpenen angereichert ist, ausgehend von der im Schritt (ii) erhaltenen Zellkultur.

2. Verfahren zur Herstellung eines angereicherten Rohextrakts nach Anspruch 1, wobei der Elizitationscocktail ferner mindestens einen Zelldifferenzierungsfaktor der Pflanzenzellen und mindestens ein Vorprodukt des Synthesewegs der Terpene umfasst.

3. Verfahren zur Herstellung eines angereicherten Rohextrakts nach Anspruch 2, wobei der mindestens eine Zelldifferenzierungsfaktor der Pflanzenzellen unter einem Zytokin, einem Gibberillin und einer Mischung davon ausgewählt wird.

4. Verfahren zur Herstellung eines angereicherten Extrakts nach Anspruch 3, wobei der mindestens eine Zelldifferenzierungsfaktor der Pflanzenzellen unter Benzylaminopurin (BAP), 6-γ,γ-Dimethylallylaminopurin (2iP) und einer Mischung davon ausgewählt wird.

5. Verfahren zur Herstellung eines angereicherten Rohextrakts nach einem der Ansprüche 2 bis 4, wobei das mindestens eine Synthesevorprodukt der Terpene in der Gruppe ausgewählt wird, die aus Natriumpyruvat, Kaliumpyrophosphat und einer Mischung davon besteht.

6. Verfahren zur Herstellung eines angereicherten Rohextrakts nach einem der Ansprüche 1 bis 5, das ferner einen Schritt (iv) des Erhaltens eines gereinigten Extrakts des pentacyclischen Triterpens ausgehend von dem im Schritt (iii) erhaltenen angereicherten Rohextrakt umfasst.

## Claims

1. A process for producing a crude extract enriched in a pentacyclic triterpene, which is celastrol, comprising the following steps:
(i) a phase of proliferation of cells of a plant of the Celastraceae family, which is *Tripterygium wilfordii,* in a proliferation medium,
(ii) a phase of elicitation by adding an elicitation cocktail to the cell culture obtained in step (i), said elicitation cocktail comprising at least one elicitor of monocarboxylic compound type, which is methyl jasmonate, and at least one biotic elicitor, which is chitin, and
(iii) preparation of a crude extract enriched in pentacyclic tripterpenes from the cell culture obtained in step (ii).

2. The process for producing an enriched crude extract according to claim 1, wherein the elicitation cocktail further comprises at least one cell differentiation factor for plant cells and at least one precursor of the terpene synthesis pathway.

3. The process for producing an enriched crude extract according to claim 2, wherein the at least one cell differentiation factor for plant cells is selected from a cytokine, a gibberellin and a mixture thereof.

4. The process for producing an enriched crude extract according to claim 3, wherein the at least one cell differentiation factor for plant cells is selected from benzylaminopurine (BAP), 6-γ,γ-dimethylallylaminopurine (2iP) and a mixture thereof.

5. The process for producing a crude extract enriched according to any one of claims 2 to 4, wherein the at least one precursor of terpene synthesis is selected from the group consisting of sodium pyruvate, potassium pyrophosphate and a mixture thereof.

6. The process for producing an enriched crude extract according to any one of claims 1 to 5, further comprising a step (iv) of obtaining a purified extract of the pentacyclic triterpene from the enriched crude extract obtained in step (iii).
